## Europäisches Patentamt
## European Patent Office
## Office européen des brevets

(11) Veröffentlichungsnummer: **0 325 805**
**A2**

# (12) EUROPÄISCHE PATENTANMELDUNG

(21) Anmeldenummer: **88121916.6**

(51) Int. Cl.⁴: **A61B 7/04**

(22) Anmeldetag: **30.12.88**

(30) Priorität: **31.12.87 DE 3744605**

(43) Veröffentlichungstag der Anmeldung:
**02.08.89 Patentblatt 89/31**

(84) Benannte Vertragsstaaten:
**DE ES FR GB IT**

(71) Anmelder: **Heimann, Jochen**
**Leonhardistrasse 10a**
**D-8011 Siegertsbrunn(DE)**

(72) Erfinder: **Heimann, Jochen**
**Leonhardistrasse 10a**
**D-8011 Siegertsbrunn(DE)**

(74) Vertreter: **Schmitz, Hans-Werner, Dipl.-Ing. et al**
**Schoppe . Schmitz . Weber Patentanwälte**
**Ludwig-Ganghofer-Strasse 20**
**D-8022 Grünwald bei München(DE)**

(54) **Messwertaufnehmer zur nichtinvasiven Messung von Schall, Druck und Vibrationen am menschlichen Körper.**

(57) Die Erfindung betrifft einen Meßwertaufnehmer (1) zur nicht-invasiven Messung von Schall, Druck und Vibrationen am menschlichen Körper, der zur Erreichung einer sehr guten Ansprechcharakteristik und eines geringen Gewichtes und geringer Abmessungen einen Wandler in Form einer Wandlermembran (23) aufweist. Vorzugsweise ist die Wandlermembran (23) als piezoelektrische Folie ausgebildet, die die mechanischen Eingangsgrößen in elektrische Signale umwandelt, die mit Hilfe von Auswerteinheiten weiterverarbeitet werden können.

FIG.2

EP 0 325 805 A2

Die Erfindung geht aus von einem Meßwertaufnehmer zur nichtinvasiven Messung von Schall, Druck und Vibrationen am menschlichen Körper nach dem Oberbegriff des Anspruchs 1.

Ein solcher Meßwertaufnehmer ist aus der Broschüre "Kreislaufkontrolle mit dem Infraton-Pulsabnehmer" (System: Boucke-Brecht) bekannt. Dieser Meßwertaufnehmer weist einen Fühlstift auf, der auf einen in einem Gehäuse angeordneten Spezialkondensatorwickel mit einem Wickelkern wirkt. Der Kondensatorkern ist mit einem inneren Gummimantel und einem darin befindlichen Luftpolster umgeben. Um den Gummimantel herum ist ein Silberdrahtgewebe angeordnet, um dessen Außenfläche wiederum ein äußerer Gummimantel angeordnet ist.

Dieser Meßwertaufnehmer stellt einen hochkapazitiven elektrostatischen Meßwandler dar, der beispielsweise durch Binden oder Bandagen an den entsprechenden Stellen des menschlichen Körpers zur Pulsabnahme befestigt werden kann.

Andere bekannte Meßwertaufnehmer, insbesondere solche für Herzschallmessungen, besitzen einen Fühlstift in Verbindung mit einem Hebelmechanismus zur Übertragung der Fühlerauslenkung auf einen mechano-elektrischen Wandler.

Derartige Meßwertaufnehmer sind vor allem deswegen nachteilig, weil sie groß und relativ schwer sind und daher dem Patienten im an der Körperoberfläche angebrachten Zustand hinderlich sind. Somit sind Messungen praktisch nur im Ruhezustand des Patienten möglich, was wiederum den Anwendungsbereich der bekannten Meßwertaufnehmer einschränkt, da Messungen unter Belastung, also beispielsweise bei sich bewegenden Patienten fast nicht möglich sind, bzw. mit erheblichen Behinderungen der Person einhergehen.

Est ist daher Aufgabe der vorliegenden Erfindung, einen Meßwertaufnehmer der im Oberbegriff des Anspruchs 1 angegebenen Art zu schaffen, der leicht ist und dessen Abmessungen gering sind, um zu verhindern, daß er bei der Messung hinderlich ist.

Die Lösung dieser Aufgabe erfolgt durch die Merkmale des Anspruchs 1.

Durch die Ausbildung des Wandlers als Wandlermembran wird eine äußerst kompakte und leichte Bauform des erfindungsgemäßen Meßwertaufnehmers möglich, was dazu führt, daß der Meßwertaufnehmer in am menschlichen Körper angebrachten Zustand Probanden in keiner Weise hinderlich ist. Somit ist es mit dem erfindungsgemäßen Meßwertaufnehmer problemlos möglich, auch Messungen im belasteten Zustand des Probanden, also beispielsweise unter Ausführung von Bewegungen unterschiedlicher Art, durchzuführen.

Dabei ergeben sich aufgrund des geringen Gewichtes sehr gute Meßcharakteristika, die bei dem erfindungsgemäßen Meßwertaufnehmer zu sehr genauen Meßergebnissen führen.

Hierzu sind im Vorfeld zur Erzielung von Auslegungs- und Konstruktionskriterien experimentelle Untersuchungen durchgeführt worden. Berührungslose Messungen mit einer Laser-Doppler-Anordnung haben ergeben, daß bei Schall-und Vibrationsmessungen die Masse des Meßwertaufnehmers die zu messende Größe der Auslenkung an der Körperoberfläche verfälscht. Optimierte Meßsignale lassen sich hinsichtlich des frequenzabhängigen Amplitudenganges als auch der Meßsignal-Bandbreite gewinnen, wenn man neben reduzierter Masse des Meßwertaufnehmers gleichzeitig die Größe des Auflagedruckes des Meßwertaufnehmers auf der Meßstelle berücksichtigt. Es hat sich bei weiteren, im Rahmen der Erfindung durchgeführten Untersuchungen ge zeigt, daß die in der klassischen Literatur enthaltenen Angaben über die Höhe des Anpreßdruckes des Meßwertaufnehmers zu mechanischen Belastungen der Meßstelle führen, so daß dadurch eine nichtlineare, frequenzabhängige Dämpfung der Meßgröße am Meßort entsteht. Mit Meßwertaufnehmern, die hohe Eigenmasse aufweisen, und mit Meßanordnungen, die zu hohem Auflagedruck führen, was teilweise durch die hohe Eigenmasse der Meßwertaufnehmer oder durch gespannte Bänder und Gurte erzeugt wird, die zur Fixierung der Meßwertaufnehmer dienen, lassen sich keine zuverlässigen diagnostischen Aussagen über Herzgeräusche tätigen, die im höherfrequenten Anteil des Herzschallsignales enthalten sind.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung von Ausführungsbeispielen anhand der Zeichnungen.

Es zeigt:

Fig. 1 ein Blockschaltbild unter Verwendung eines erfindungsgemäßen Meßwertaufnehmers;

Fig. 2 eine schematisch leicht vereinfachte auseinandergezogene Schnittdarstellung einer ersten Ausführungsform des bei der Meßkette gemäß Fig. 1 verwendbaren Meßwertaufnehmers;

Fig. 3 eine Seitenansicht einer zweiten Ausführungsform des bei der Meßkette gemäß Fig. 1 verwendbaren Meßwertaufnehmers; und

Fig. 4 eine Draufsicht auf den Meßwertaufnehmer gemäß Fig. 3 aus Richtung des Pfeiles IV in Fig. 3.

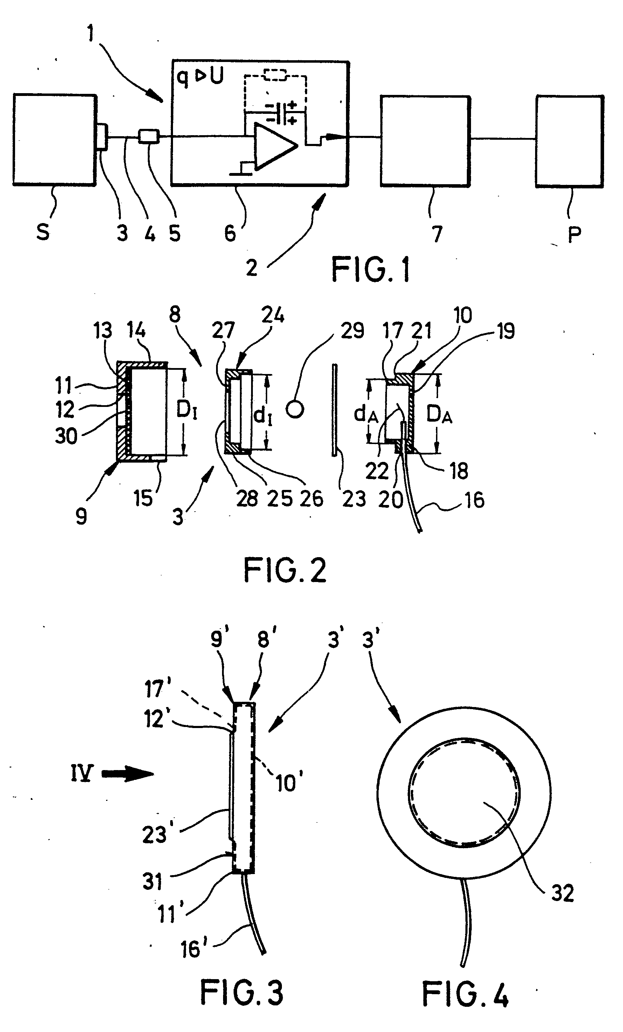

In Fig. 1 ist schematisch vereinfacht ein Meßwertaufnehmer 1 gemäß vorliegender Erfindung dargestellt, der ein Teil einer Meßkette 2 bildet. Der Meßwertaufnehmer 1 weist gemäß Fig. 1 eine Sensorzelle 3 auf, die an einem zu vermessenden System S angebracht ist, das beispielsweise der menschliche Körper zur Erfassung von Schall, wie beispielsweise Herzschall, Druck und Vibrationen sein kann. Der Meßwertaufnehmer 1 kann am menschlichen Körper S beispielsweise durch eine körperverträgliche leicht klebende Substanz angebracht werden, da er extrem leicht und klein ist. Die Sensorzelle 3 ist über ein sehr kurzes Kabel 4 und eine Steckverbindung 5 mit einem aktiven Ladungsverstärker 6 verbunden. Der aktive Ladungsverstärker, der die Funktion eines Signalvorverstärkers erfüllt, ist an seinem Ausgang über ein längeres Kabel mit einer Auswerteeinheit 7 verbunden, die wiederum mit einer Registrier- und Ausgabeeinheit, wie beispielsweise einem Schreiber 8, verbunden sein kann.

In der zuvor beschriebenen Meßkette 2 wird der Meßwertaufnehmer 1 von der im folgenden detailliert zu beschreibenden Sensorzelle 3, dem Kabel 4, das eine Übertragungseinrichtung bildet, und dem Signalvorverstärker 6 gebildet.

Bei zuvor beschriebener Anordnung sollte das Kabel 4 möglichst kurz sein, wobei im Extremfall die Kabellänge zu Null werden kann, was bedeutet, daß der Vorverstärker 6 in oder an der Sensorzelle 3 angeordnet wird.

Ferner sollte das Kabel 4 einen hohen Isolationswiderstand und eine geringe Kapazität haben, damit geringe dielektrische Verluste auftreten. Vorzugsweise ist das Kabel 4 als Koaxialkabel auszubilden, was auch aus schirmungstechnischen Gründen Vorteile ergibt.

Die Übertragungseinrichtung 4 und der Vorverstärker 6 lassen sich weiterhin zu einer drahtlosen Übertragungseinrichtung zusammenfassen. Dies hat den Vorteil, daß in einigen Anwendungen, wie Langzeit- und/oder Intensiv-Überwachung von Patienten im mobilen, wie auch im stationären Betrieb das Tragen von Verbindungskabeln entfällt.

Die drahtlose Übertragungseinrichtung läßt sich als Hochfrequenz-Sender ausführen, dessen Trägersignal mit dem Meßsignal moduliert wird. Hierbei sollten jedoch Maßnahmen zur Selektivität enthalten sein, um z. B. die von einzelnen Patienten gewonnenen Signale über einen zentralen Empfänger empfangen und kanalspezifisch separieren zu können. Es bietet sich daher ein zusätzlicher hochfrequenter Zwischenträger oder in Ausnutzung der Vorteile einer digitalen Signalübertragung die Einrichtung einer kanalselektiven Codierung an. Derartige Einrichtungen zur Codierung von Signalen sind in der Nachrichtenübertragungstechnik hinreichend beschrieben.

In einer anderen Ausführung kann die drahtlose Übertragungseinrichtung durch eine Infrarot- oder Laser-Sende-einrichtung realisiert werden. Auch hier gelten die im vorherigen Abschnitt erläuterten Maßnahmen zur kanalspezifischen Selektierung der einzelnen Patienten-Meßwerte. Eine digitale Signalübertragung ist in diesem Falle von besonderem Vorteil, da über eine geeignete Wahl des Puls/Pausen-Verhältnisses der digitalen Pulssignale eine erhebliche Energieeinsparung gelingt. Technisch realisierbar sind hier Taktverhältnisse von 1:10 bis 1:100 in Abhängigkeit von der technischen Ausführung der Infrarot- oder Laser-Übertragungseinrichtung und der Größe der Senderreichweite.

Ist man auf eine geringe Energieversorgung angewiesen, lasesn sich die digitalen Sensordaten auch über Lichtleitfasern übertragen. Unter Berücksichtigung der zuvor beschriebenen Maßnahmen der Mehrfachausnutzung einer Übertragungseinrichtung können die am Patienten angelegten Sensoren über eine Übertragungseinrichtung entweder durch einen Hochfrequenzsender und/oder eine optische Übertragungseinrichtung drahtlos oder über ein Kabel oder eine Lichtleitfaser übertragen werden. Hierbei übernimmt eine Modulationseinrichtung das Aufschalten der einzelnen Sensorsignale vor der Übertragungseinrichtung.

Für eine digitale Übertragung der Sensorsignale müssen die analogen Meßsignale des Wandlers in digitale Signale des Wandlers umgesetzt werden. Derartige Verfahren sind in der technischen Literatur hinreichend beschrieben. Hierbei ist eine serielle Ausgabe der digitalen Werte des Umsetzers vorteilhaft, da eine parallele Ausgabe einen zusätzlichen Aufwand an Übertragungseinrichtungen erfordert.

Vorzugsweise werden der Signalvorverstärker 6 und der Analog/Digitalwandler in einer Funktionseinheit zusammengefaßt. Dieses läßt sich unter anderem z. B. durch einen Ladungs-Frequenz-Umsetzer realisieren, bei dem die Ladungsmenge des mechano-elektrischen Wandlers direkt in ein frequenz-proportionales Signal umgesetzt wird.

Im folgenden wird anhand von Fig. 2 eine erste Ausführungsform der in Fig. 1 dargestellten Sensorzelle 3 beschrieben.

Die Sensorzelle 3, die in Fig. 2 in auseinandergezogener Darstellung gezeigt ist, weist ein Gehäuse 8 auf, das aus einem elektrisch leitenden Gehäuseteil 9 und einem elektrisch nicht leitenden Gehäuseteil 10 aufgebaut ist.

Wie aus Fig. 2 ersichtlich ist, ist das elektrisch leitende Gehäuseteil 9 als rotationssymmetrisches topfförmiges Teil ausgebildet. Es weist in seiner Frontplatte 11 eine zentrische Ausnehmung 12 auf. Auf der Innenseite der Frontplatte 11 ist ein Federglied 13 in Form einer elastischen Membran befestigt, das die Ausnehmung 12 vollständig dichtend abdeckt. Das Gehäuseteil 9 weist ferner in seiner umlaufenden Ringwand 14 eine Ausnehmung 15 auf, durch die ein Anschlußdraht 16 im montierten Zustand der Sensorzelle 3 hindurchgeführt wer-

den kann. Da das Gehäuseteil 9 als elektrisch leitendes Teil ausgebildet ist, kann es vorzugsweise aus Messing bestehen. Die Oberfläche des Gehäuseteils 9 muß zusätzlich vergütet werden, um gegen Desinfektions- und Reinigungsmittel resistent zu sein. Eine derartige Oberflächenveredelung kann durch Vernickeln oder Verchromen erreicht werden.

Das zweite aus elektrisch nich-leitendem Material bestehende Gehäuseteil 10 kann beispielsweise aus PVC ausgebildet sein. Es ist jedoch ein Kunststoff zu verwenden, dessen mechanische und chemische Eigenschaften durch Reinigungs- und Desinfektionsmittel nicht verändert werden. Zudem sollte dieser Kunststoff eine gewisse Temperaturbeständigkeit aufweisen, so daß auf jeden Fall keine Ausgasung stattfindet, die den Patienten beeinträchtigen könnte. Im einzelnen weist das Gehäuseteil 10 ebenfalls eine rotationssymmetrische Form mit einem Außenmesser $D_A$ auf, der je nach Passungsart dem Innendurchmesser $D_I$ des Gehäuseteiles 9 entspricht oder etwas größer ist. Ferner weist das Gehäuseteil 10 einen ringförmigen Bund 17 auf, dessen Außendurchmesser $d_A$ kleiner ist als der Außendurchmesser $D_A$, wohingegen die Innendurchmesser der beiden Teile gleich sind. In einer frontseitigen Außenwand 18 des Gehäuseteiles 10 ist eine Ausnehmung 19 vorgesehen, die als Druckausgleichsöffnung dient. Das Kabel 16 wird duch eine Ausnehmung 20 in einem Ringwandbereich 21 des Gehäuseteiles 10 in dessen Innenbereich geführt und dort fixiert. Hierbei wird ein blankes Ende 22 so angeordnet, daß dieses elektrisch leitend mit einem Wandler in Form einer Wandlermembran 23 z. B. durch Klebung verbunden werden kann.

Die Wandlermembran 23 ist vorzugsweise als piezoelektrische Folie ausgebildet, die z. B. aus einem Kunststoffträger bestehen kann, der beidseitig mit einer elektrisch leitenden Schicht bedampft ist. Wie Fig. 2 verdeutlicht, ist die Folie im unmontierten Zustand im wesentlichen plan und ebenfalls rotationssymmetrisch, vorzugweise kreisförmig ausgebildet. Die Montage der Folie 23 wird im folgenden näher beschrieben werden.

Die Sensorzelle 3 weist ferner einen ebenfalls rotationssymmetrisch ausgebildeten Klemmring 24 auf, der im montierten Zustand mit dem Gehäuseteil 10 zusammenwirkt. Der Klemmring 24 weist einen Basisbereich 25 mit gegenüber einem Ringbereich 26 größerer Wandstärke auf. Der Ringbereich 26 weist einen Innendurchmesser $d_i$ auf, der im wesentlichen dem Außendurchmesser $d_A$ des Ringbereiches 17 des Gehäuseteiles 10 entsprechen kann. Da die Membran 23 zwischen dem Klemmring 24 und dem Gehäuseteil 10 eingespannt wird, ist es möglich, durch entsprechende Ausbildung der Flanken des Bereiches 26 und des Bereiches 17 unterschiedlichen Vorspannungen der Membran 23 im Zuge der Montage bzw. des Einspannens zu erreichen. Das bedeutet mit anderen Worten, daß bei engen Toleranzen eine hohe Vorpsannung erzielt wird, was bedeutet, daß der Innendruchmesser des Klemmringes im wesentlichen gleich dem Außendurchmesser des Bereiches 17 des Gehäuseteiles 10 ist, wohingegen bei größer werdendem Innendurchmesser des Klemmringes die Vorspannung abnimmt. Diese Montage-Parameter sind deshalb in dieser Form wählbar, da die Wanndlermembran 23 nur einige Mikrometer stark ist. Darüber hinaus ist es zur Verfeinerung der Einstellung möglich, die entsprechenden aufeinandergleitenden Bereiche des Klemmringes bzw. des Gehäuseteiles 10 anzuphasen, d. h. mit anderen Worten, ihre Steilheit unterschiedlich auszu- bilden.

Der Klemmring 24 weist ferner in seiner im montierten Zustand auf der Membran 13 aufliegenden Fläche 27 eine kreisförmige Durchtrittsausnehmung 28 auf.

Schließlich weist die Sensorzelle 3 einen im Ausführungsbeispiel kugel- bzw. ellipsoidförmigen Fühler 29 auf. Der Fühler 29 dient zur Erfassung der mechanischen Eingangsgröße und wird daher im Einsatzfall beispielsweise auf die Körperoberfläche aufgelegt.

Wird die in Fig. 2 in auseinandergezogener Darstellung gezeigte Sensorzelle 3 montiert, wird nach Anbringung der Membran 13 im Gehäuseteil 9 der Fühler 29 auf der Membran 13 zentrisch sur Ausnehmung 12 fixiert wird. Danach wird die Membran 23 mit dem Klemmring 24 auf dem Gehäuseteil 10 unter Druckaufbringung festgeklemmt. Diese Einheit wird dann in das Gehäuseteil 9 eingelet. Dadurch, daß die Membran 13 radial etwas über das Gehäuseteil 10 und den Klemmring 24 übersteht, wird beim Einlegen der Einheit (13, 10, 23) ein elektrischer Kontakt mit dem Gehäuseteil 9 hergestellt. Deshalb ist zum Schließen des elektrischen Kreises nur noch erforderlich, die Schirmungsmasse eines z. B. zu verwendenden Koaxialkabels als Übertragungseinrichtung 4 mit dem Gehäuseteil 9 elektrisch zu verbinden. Dabei wird in der zuvor beschrie benen Art und Weise sowohl die Membran 23 durch das Zusammenwirken des Klemmrings 24 mit dem Gehäuseteil 10 vorgespannt, als auch durch die Wirkung des Fühlers 29, da dieser auf der elastischen Membran 14 aufliegt. Dabei tritt der Fühler 29 und der entsprechende Teil der Membran 13 durch die Austrittsöffnung 12, so daß er im montierten Zustand der Sensorzelle 3 über die Außenfläche der Frontplatte 11 hervorsteht. Somit ist der Fühler 29 mit der unter Vorspannung stehenden Membran 23 gekoppelt.

Hierbei ist die Steifigkeit der das Federglied bildenden Membran 13 kleiner als die Steifigkeit der Wandlermembran 23. Vorzugsweise beträgt die Steifigkeit des Federgliedes 13 10 % bis 30 % der Steifigkeit der Wandlermembran. Ferner ist die Masse des Fühlers 29 wesentlich kleiner als die Gehäusemasse. Bei einer besonders bevorzugten Ausführungsform beträgt die Fühlermasse ca. 0, 02 g, wohingegen die Gehäusemasse ungefähr 1,68g beträgt. Die Bereiche zwischen Fühlermasse zu Gehäusemasse liegen bei unterschiedlichen Ausführungsformen zwischen 1:10 bis 1:30.

Die Gesamtmasse der Sensorzelle ist insgesamt sehr gering und liegt im allgemeinen unter 2 g.

Darüber hinaus ist zu bemerken, daß die Druckausgleichsbohrung wesentlich kleiner (z. B. 0,5 - 0,8 mm) ist, als die zu empfangene Wellenlänge der zu erfassenden physikalischen Größe (Schall, Druck, Vibration).

Wie beschrieben, ragt der Fühler 29 unter Abdichtung durch die Membran 13 im montierten Zustand über die Außenfläche des Gehäuses 9 hervor, so daß er aufgrund von Schall, Druck und Vibrationen erzeugte mechanische Bewegungen beispielsweise der Körperoberfläche erfassen kann. Diese erfaßten mechanischen Eingangsgrößen werden vom Fühler 29 auf die den Wandler bildende piezoelektrische Membran 23 übertragen, die wiederum die mechanische Eingangsgröße in ein elektrisches Signal umwandelt. Das elektrische Signal wird über die Übertragungseinrichtung z. B. in Form des Kabels 4 zum Signalvorverstärker 6 geleitet, der eine Signalverstärkung durch die beispielhaft in Fig. 1 dargestellte Schaltungsanordnung, auf die aus Offenbarungsgründen ausdrücklich hingewiesen wird, vornimmt.

In der Auswerteeinheit 7 und dem Schreiber P können die erfaßten Signale dann meßtechnisch ausgewertet und ausgegeben werden.

Im folgenden wird anhand der Fig. 3 und 4 ein zweites Ausführungsbeispiel einer Sensorzelle 3′ beschrieben.

Die Sensorzelle 3′ weist ebenfalls ein elektrisch leitendes Gehäuseteil 9′ und ein elektrisch nichtleitendes Gehäuseteil 10′ auf. Das Gehäuseteil 9′ ist im wesentlichen genauso aufgebaut wie das Gehäuseteil 9, weist jedoch einen wesentlich größeren Außendurchmesser und eine Durchtrittsausnehmung 12′ ebenfalls mit wesentlich größerem Durchmesser auf als die entsprechenden Durchmesser der Ausführungsform gemäß Fig. 2. Die Ausbildung des Gehäuseteiles 10′ entspricht im wesentlichen demjenigen des Gehäuseteiles 10, so daß auf die dementsprechenden Ausführungen im Hinblick auf die Ausführungsform gemäß Fig. 2 Bezug genommen werden kann.

Die Sensorzelle 3′ weist ferner ebenfalls eine Wandlermembran 23′ auf, die vorzugsweise ebenfalls als piezoelektrische Folie ausgebildet ist. Die Wandlermembranfolie 23′ wird mit einem in das Gehäuse 8′ geführten Verbindungskabel 16′ kontaktiert. Wie aus Fig. 4 ersichtlich ist, ist auch die Sensorzelle 3′ rotationssymme trisch ausgebildet, wobei im montierten Zustand die Wandlermembran 23′ über die Außenfläche 31 des leitenden Gehäuseteiles 9′ vorsteht. Hierbei wird die Vorspannung ebenfalls durch eine Klemmwirkung zwischen den Gehäuseteilen 9′ und 10′ erreicht, wobei der entsprechende umlaufende Bund 17′ des nicht-leitenden Gehäuseteiles 10′ im montierten Zustand durch die Durchgangsöffnung 12′ hindurchtritt und über die Fläche 31 hinausragt. Mithin ist der entsprechende Bereich 32 der Wandlermembran 23′ im montierten Zustand der Sensorzelle 3′ gespannt und im Abstand zur Fläche 31 angeordnet, so daß er im Unterschied zur Ausführungsform gemäß Fig. 2 sowohl den Fühler als auch den Wandler bildet. Dies ergibt den Vorteil einer besonders einfachen und dabei trotzdem hochempfindlichen Ausbildung der Sensorzelle 3′. Denn es ist möglich, einen separaten Klemmring, ein zusätzliches der Vorspannung dienendes Federglied wie auch einen separaten Fühler zu vermeiden. Dementsprechend kann die Sensorzelle 3′ gegebenenfalls noch leichter ausgebildet werden. Andererseits ist es aber möglich, im Bedarfsfall zusätzlich einen Klemmring gemäß Fig. 2 zu verwenden.

Bezüglich aller übereinstimmender Details und der Funktionsweise kann auf die Beschreibung der Sensorzelle 3, die in Fig. 2 dargestellt ist verwiesen werden.

Obwohl im vorangehenden eine Messung am menschlichen Körper beschrieben wurde, ist der erfindngsgemäße Meßwertaufnehmer auch für andere zu vermessende Systeme, wie z. B. Maschinen, geeignet.

**Ansprüche**

1. Meßwertaufnehmer (1) zur nicht-invasiven Messung von Schall, Druck und Vibrationen am menschlichen Körper
mit einer Sensorzelle (3; 3′), die einen Fühler (29; 23′) aufweist, der zur Erfassung von mechanischen Eingangsgrößen auf die Körperoberfläche auflegbar ist,
mit einem Wandler (23; 23′), der mit dem Fühler (29; 23′) gekoppelt ist und die mechanische Eingangsgröße in ein elektrisches Signal umwandelt,
mit einer Übertragungseinrichtung (4), die mit dem Wandler (23; 23′) verbunden ist, und
mit einem Signalvorverstärker (6), der über die Übertragungseinrichtung (4) mit dem Wandler (23; 23′) verbunden ist, wobei der Signalvorverstärker (6) an eine Auswerteeinheit (7, P) anschließbar ist, dadurch gekennzeichnet,
daß der Wandler eine Wandlermembran (23; 23′) ist.

2. Meßwertaufnehmer nach Anspruch 1, dadurch gekennzeichnet, daß die Wandlermembran (23; 23´) eine piezoelektrische Folie ist.

3. Meßweraufnehmer nach Anspruch 1 oder 2, dadurch gekennzeichnet, daß der Wandler (23; 23´) in einem Gehäuse (8; 8´) angeordnet ist.

4. Meßwertaufnehmer nach Anspruch 3, dadurch gekennzeichnet, daß das Gehäuse (8; 8´) aus einem elektrisch leitenden und elektrisch nicht-leitenden Gehäuseteil (9, 10; 9´, 10´) besteht.

5. Meßwertaufnehmer nach Anspruch 4, dadurch gekennzeichnet, daß das Gehäuse (8; 8´) rotationssymmetrisch ist.

6. Meßwertaufnehmer nach einem der Ansprüche 1 bis 5, dadurch gekennzeichnet, daß der Fühler (29; 23´) durch eine Wandung (11; 11´) des Gehäuses (8; 8´) hindurchtritt und diese überragt.

7. Meßwertaufnehmer nach einem der Ansprüche 1 bis 6, dadurch gekennzeichnet, daß die Fühlermasse wesentlich kleiner ist als die Gehäusemasse.

8. Meßwertaufnehmer nach Anspruch 7, daduch gekennzeichnet, daß die Aufnehmergesamtmasse sehr gering ist und vorzugsweise unter 2 g beträgt.

9. Meßwertaufnehmer nach einem der Ansprüche 1 bis 8, dadurch gekennzeichnet, daß die Übertragungseinrichtung ein kurzes Kabel (4) ist.

10. Meßwertaufnehmer nach Anspruch 9, dadurch gekennzeichnet, daß die Kabel (4) einen hohen Isolationswiderstand und eine geringe Kapazität aufweist.

11. Meßwertaufnehmer nach Anspruch 4, dadurch gekennzeichnet, daß das elektrisch leitende Gehäuseteil (9; 9´) aus Messing besteht.

12. Meßwertaufnehmer nach Anspruch 4, dadurch gekennzeichnet, daß das elektrisch nicht-leitende Gehäuseteil (10; 10´) aus PVC besteht.

13. Meßwertaufnehmer nach einem der Ansprüche 1 bis 12, dadurch gekennzeichnet, daß der Signalvorverstärker ein aktiver Ladungsverstärker (6) ist.

14. Meßwertaufnehmer nach einem der Ansprüche 1 bis 13, dadurch gekennzeichnet, daß der Signalvorverstärker so nahe wie möglich, im Extremfall im bzw. am gehäuse (8; 8´) angeordnet ist.

15. Meßwertaufnehmer nach einem der Ansprüche 1 bis 14, dadurch gekennzeichnet, daß im Gehäuse (8; 8´) eine Druckausgleichsbohrung (19) vorgesehen ist, die wesentlich kleiner ist als die zu empfangende Wellenlänge.

16. Meßwertaufnehmer nach einem der Ansprüche 1 bis 15, dadurch gekennzeichnet, daß der Fühler (29) mittels eines Federgliedes (13) mit dem Wandler (23) gekoppelt ist.

17. Meßwertaufnehmer nach Anspruch 16, dadurch gekennzeichnet, daß die Steifigkeit des Federgliedes (13) kleiner ist als die der Wandlermembran (23).

18. Meßwertaufnehmer nach Anspruch 16 oder 17, dadurch gekennzeichnet, daß das Federglied (13) eine elastische Membran ist.

19. Meßwertaufnehmer nach einem der Ansprüche 1 bis 18, dadurch gekennzeichnet, daß der Fühler (29) kugelförmig oder ellipsoidförmig ist.

20. Meßwertaufnehmer nach einem der Ansprüche 1 bis 15, dadurch gekennzeichnet, daß Fühler und Wandler ein Teil (23´) sind.

21. Meßwertaufnehmer nach Anspruch 20, dadurch gekennzeichnet, daß das den Fühler und den Wandler bildende Teil eine piezoelektrische Wandlermembran (23; 23´) ist.

22. Meßwertaufnehmer nach Anspruch 20 oder 21, dadurch gekennzeichnet, daß die Membran (23´) zwischen den Gehäuseteilen (9´; 10´) eingespannt ist.

23. Meßwertaufnehmer nach einem der Ansprüche 1 bis 8 und 11 bis 22, dadurch gekennzeichnet, daß die Übertragungseinrichtung (4) eine drahtlose Übertragungseinrichtung, bestehend aus einem modulierten Hochfrequenzsender oder einem optischen Sender, bestehend aus einer Infrarot- oder Laserübertragungsstrecke, ist.

q ▷ U

1

S    3  4  5    6

2

7    P

**FIG.1**

13 14  8    27 24    29  17 21  10    19

11

12

30

$D_I$

$d_I$

$d_A$    $D_A$

9    15    3    28 25 26    23 20    22    18

16

**FIG.2**

9'  8'    3'    3'

17'

12'

IV

10'

23'

31

11'

32

16'

**FIG.3**    **FIG.4**